# EUROPEAN PATENT SPECIFICATION

(11) **EP 1 665 978 B1**
(45) Date of publication and mention of the grant of the patent: **08.03.2017**
(21) Application number: 04773163.3
(22) Date of filing: 16.09.2004
(51) Int. Cl.: A61B 1/12

(54) **ENDOSCOPE**
ENDOSKOP
ENDOSCOPE

(30) Priority: 19.09.2003 JP 2003328939
(43) Date of publication of application: 07.06.2006
(73) Proprietor: Olympus Corporation, Tokyo 192-8507 (JP)
(72) Inventor: MIYOSHI, Hiroaki, Hachioji-shi, Tokyo 192-8512 (JP)
(74) Representative: von Hellfeld, Axel
(86) International application number: PCT/JP2004/013504
(87) International publication number: WO 2005/027738

(56) References cited:
- JP-A- 6 154 155
- JP-A- 6 154 155
- JP-A- 8 140 929
- JP-A- 11 104 070
- JP-A- 2001 137 175
- JP-U- H0 253 701
- JP-Y2- H 074 801
- US-B1- 6 217 510

## Description

### Technical Field

The present invention relates to an endoscope having a plurality of solid-state image pick-up devices for observation with normal light and for observation with specific light.

### Background Art

Recently, an endoscope is widely used in the medical field.

US 6,217,510 B1, on which document is based the preamble of claim 1, discloses an endoscope apparatus comprising an endoscope with an insertion part presenting, at the distal end portion: an object window, an object lens and an image sensor for usual observation; an object window, an object lens and an image guide fiber for fluorescent observation; a monitor connected downstream of the image sensor and the image guide fiber; two illumination windows; a nozzle for discharging fluid to the object windows is also provided; and a forceps hole for permitting a forceps to protrude from the insertion part.

JP 6 154 155 A discloses an endoscope apparatus comprising an endoscope with an insertion part presenting, at the distal end portion: a first and a second observation windows; two illumination-light study systems; an air and water supply nozzle; and treatment implement channel mouth.

Generally, the body fluid or the like is adhered to the outer surface of an objective lens system of the endoscope and this prevents the observation, upon inserting the endoscope in the body cavity. Therefore, an air/water feed nozzle for cleaning is arranged. The air/water feed nozzle discharges cleaning water or sprays air, thereby ensuring the clear field of view for observation of the objective lens system.

For example, according to a first conventional art, Japanese Unexamined Utility Model Application Publication No. 1-133901 discloses an endoscope having a plurality of objective lens systems, wherein a plurality of air/water feed nozzles are provided to ensure the fields of view for observation of the objective lens systems.

Further, according to the first conventional art, one nozzle has two discharge directions, thus to ensure the fields of view for observation of two facing objective lens systems.

In addition, according to a second conventional art, Japanese Unexamined Patent Application Publication No. 64-24215 discloses a stereoscopic endoscope comprising a master endoscope having a first objective lens system and a slave endoscope having a second objective lens system, wherein the slave endoscope is inserted into a channel of the master endoscope for stereoscopic observation, thereby enabling the stereoscopic observation with the first objective lens system of the master endoscope and the second objective lens system of the slave endoscope.

Further, according to the second conventional art, in the stereoscopic endoscope; one nozzle is arranged to be positioned on the straight line connecting the first and second objective lens systems, and the two first and second objective lens systems are cleaned and drained off.

According to the first conventional art, one nozzle sprays the air and water in a plurality of directions and therefore its structure is complicated. Further, according to the second conventional art, the stereoscopic observation is performed by inserting the slave endoscope into the channel of the master endoscope. In this case, lens systems of both the master and slave endoscopes are equally formed and therefore the stereoscopic observation is not preferably performed.

The first and second conventional arts do not meet the needs of the endoscope which easily diagnoses, on the observation with the fluorescent light, based on information on the affected part on the observation with the normal light, whether or not the affected part is the lesion tissue.

That is, in order to meet the needs, substantially, the observation with the normal light and the observation with the fluorescent light must be switched.

According to another conventional art, an endoscope is disclosed for both the observation with the normal light and the observation with the fluorescent light. However, the insertion portion does not have the thinner diameter.

It is therefore an object of the present invention to provide an endoscope which has thinner diameter of an insertion portion, and in which the observation with normal light, the observation with fluorescent light and the like are switched, thereby making it possible to pick up endoscope images.

It is another object of the present invention to provide an endoscope capable of picking up endoscope images of different diagnostic functions by switching.

### Disclosure of Invention

### Means for Solving the Problem

The present invention is defined in independent claim 1.

### Brief Description of the Drawings

Figs. 1 to 7B relate to an embodiment 1 of the present invention, Fig. 1 is a diagram showing the entire configuration of an endoscope system having an endoscope according to the embodiment 1 of the present invention;
Fig. 2 is a cross-sectional view showing the internal structure of a distal end portion of the endoscope;
Fig. 3 is a front view showing the arrangement of an objective lens system and the like at the distal end portion of the endoscope;
Fig. 4 is a diagram showing the arrangement of two objective lens systems in one of the four areas which are obtained by division with the straight line connecting the center of the tip opening of a channel and the center of an illumination lens;
Fig. 5A is a diagram showing the arrangement in which a line segment connecting the centers of two illumination lenses crosses a line segment connecting the centers of two objective lens systems,
Fig. 5B is a diagram showing a modification of the arrangement of Fig. 5A in an exemplary manner.
Fig. 6 is a diagram showing the arrangement of the center of the channel tip opening at a specific area of six areas obtained by division with straight lines in the up, down, left, and right passing through the centers of the two objective lens systems; and
Fig. 7A is a diagram showing the arrangement in a first modification of Fig. 6 in an exemplary manner.
Fig. 7B is a diagram showing the arrangement in a second modification of Fig. 6 in an exemplary manner.

### Best Mode for Carrying Out the Invention

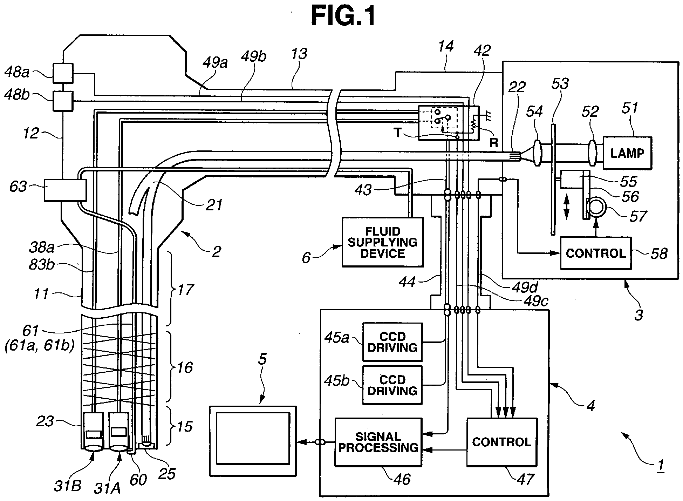

Hereinbelow, a description is given of an endoscope according to the preferable embodiment 1 of the present invention with reference to Figs. 1 to 7B.

### Embodiment 1

As shown in Fig. 1, an endoscope system 1 according to the embodiment 1 comprises an endoscope 2 for observation with normal light and for observation with fluorescent light, a light source device 3 which supplies illumination light to the endoscope 2, a video processor 4, as a signal processing device, which performs the signal processing to the endoscope 2 (hereinafter, the light source device 3 and the video processor 4 are referred to as "external device"), a monitor 5 which displays an endoscope image for observation with the normal light and an endoscope image for observation with the fluorescent light by receiving a standard video signal outputted from the video processor 4, and a fluid supplying device 6 for supplying fluid such as water or air.

The endoscope 2 comprises: an insertion portion 11 which is elongated so that it is easily inserted in the body cavity; an operating portion 12 which is arranged to the back end of the insertion portion 11; and a universal cable 13 which is extended from the side portion of the operating portion 12. The connector 14 arranged to the end portion of the universal cable 13 is detachably connected to the light source device 3.

The insertion portion 11 of the endoscope 2 comprises: a tip portion 15 which is hard and is formed to the distal end of the insertion portion 11; a bending portion 16 which is formed to the back end of the tip portion 15; and a flexible tube portion 17 reaching the front end of the operating portion 12 from the back end of the bending portion 16.

A light guide 21 for transmitting the illumination light is inserted in the insertion portion 11. The back end side of the light guide 21 is inserted in the universal cable 13 via the operating portion 12. The back end of the back end side of the light guide 21 becomes a light guide connector 22 which is projected from the connector 14.

The tip of the light guide 21 is fixed to the inside of an illumination window of a tip structuring member 23 forming the tip portion 15, and an illumination lens (illumination optical member) 25a is attached right in front of the light guide 21. The illumination light is outputted via the illumination optical member 25a. A tip cover 24 is arranged to the tip of the tip structuring member 23.

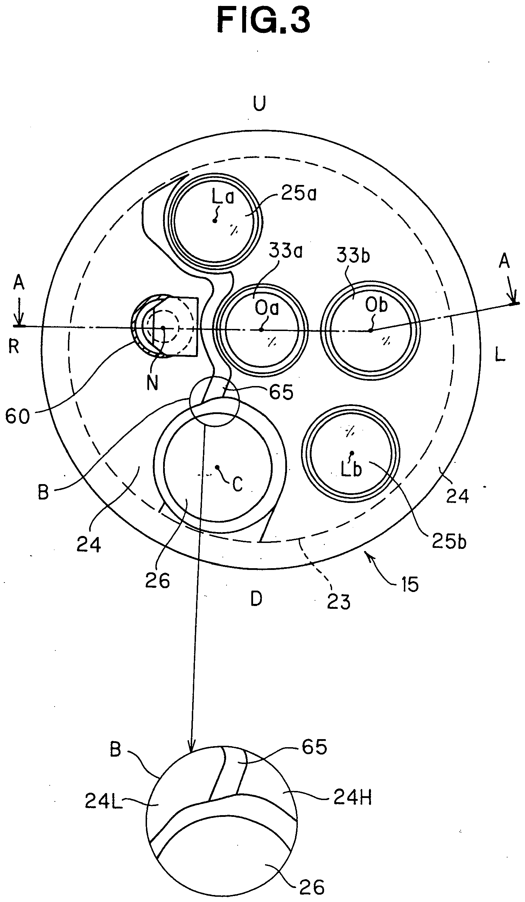

According to the embodiment, the light guide 21 is branched in the operating portion 12, and are inserted into two portions in the insertion portion 11. Referring to Fig. 3, illumination optical members 25a and 25b are arranged to the tip surface of the two divided light guides 21. The insertion portion 11 further comprises a treatment tool channel (also referred to as a clamp channel) in which a treatment tool such as a clamp (not shown in Fig. 1) is inserted. The tip of the treatment tool channel becomes a channel tip opening 26 which opens at the tip surface of the tip portion 15 as shown in Fig. 3. The treatment tool channel is branched near the back end of the insertion portion 11. One branched treatment tool channel is communicated to a treatment tool inserting port. The other branched treatment tool channel is extended to the insertion portion 11 and the universal cable 13, and is continuously communicated to a suction channel. The back end of the other channel is connected to suction means (not shown) via the connector 14.

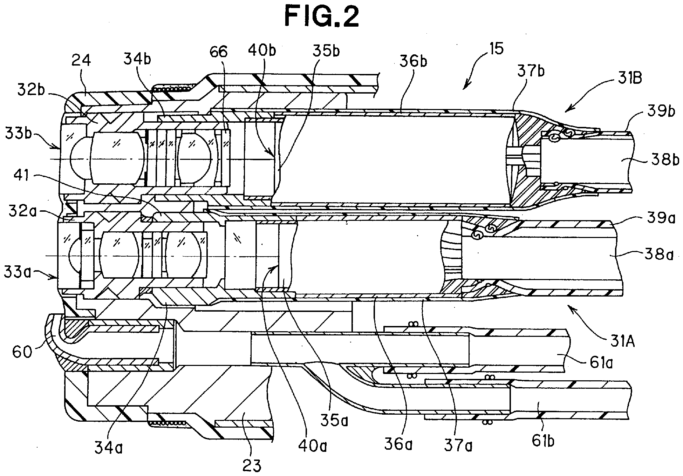

Fig. 3 shows the internal structure of the tip portion 15 shown in Fig. 2 with an A-Ob-A' cross-sectional plane. Further, referring to Fig. 3, reference numbers U, D, L, and R denote up, down, left, and right directions upon bending operation. According to the embodiment, two image pick-up units 31A and 31B having different image pick-up functions are arranged to the tip portion 15. That is, as shown in Figs. 2 and 3, an image pick-up unit (the first image pick-up portion or the first image pick-up device) 31A for observation with the normal light (observation of the subject under the first condition) and an image pick-up unit (the second image pick-up portion or the second image pick-up device) 31B for observation with the specific light, in concrete, for observation with the fluorescent light (observation of the subject under the second condition) to two image pick-up windows (observation windows) arranged to the tip structuring member 23.

Referring to Fig. 2, the image pick-up unit 31A for observation with the normal light comprises: an objective optical system (objective optical member) 33a attached to a lens frame 32a; a CCD 35a, as a solid-state image pick-up device, which is attached to a device holder 34a that is fit into the lens frame 32a; and a circuit substrate (not shown) arranged to the rear surface of the CCD 35a.

In the image pick-up unit 31A for observation with the normal light, the device holder 34a is covered with a shielding cylinder 36a, and the outside thereof is covered with a thermal contracting tube 37a.

The back end of the image pick-up unit 31A for observation with the normal light is connected to a signal cable 38a. The signal cable 38a is covered with a protecting tube 39a which prevents the short circuit of the signal cable 38a or the like. Reference number 40a denotes a light receiving surface of the CCD 35a.

The image pick-up unit 31B for observation with the fluorescent light comprises an objective optical member 33b attached to the lens frame 32b; and a CCD 35b, as a solid-state image pick-up device, which is attached to a device holder 34b that is fit to the lens frame 32b.

In the image pick-up unit 31B for observation with the fluorescent light, the device holder 34a is covered with a shielding cylinder 36b, and the outside thereof is covered with a thermal contracting tube 37b.

The back end of the image pick-up unit 31B for observation with the fluorescent light is connected to a signal cable 38b, and the signal cable 38b is covered with a protecting tube 39b. The protecting tube 39b prevents the short circuit of the signal cable 38b or the like. Reference number 40b denotes a light receiving surface of the CCD 35b.

The CCD 35b has therein an amplifying function. Therefore, as compared with a normal CCD without the amplifying function (e.g., CCD 35a used for the image pick-up unit 31A for observation with the normal light), the CCD 35b picks-up an image with a preferable S/N ratio. That is, like the case of picking-up the fluorescent image, the CCD 35b is suitable for the image pick-up operation with weak light, as compared with the observation with the normal light.

In the case of the observation with the fluorescent light, an excitation light cut-off filter 66 for cutting-off excitation light is arranged between the objective optical member 33b and the CCD 35b so as to sufficiently suppress a state in which the excitation light reflected by the living body tissue is incident on the CCD 35b of the image pick-up unit 31B for observation with the fluorescent light.

The CCD 35b has the lower thermal-tolerance, as compared with the CCD 35a, which will be described later. Referring to Fig. 3, unlike the image pick-up unit 31A for observation with the normal light, the image pick-up unit 31B for observation with the fluorescent light is arranged to the peripheral side of the tip portion 15. That is, when the heat is generated in the tip portion 15, the heat is radiated effectively on the peripheral side. Therefore, the image pick-up unit 31B for observation with the fluorescent light is arranged on the peripheral side of the tip portion 15 so as to compensate the low thermal-tolerance of the CCD 35b used for the image pick-up unit 31B for observation with the fluorescent light.

The device holder 34a is cylinder-shaped. The outer peripheral portion of the device holder 34a is cut-off on the image pick-up unit 31B for observation with the fluorescent light, thereby forming a thin portion 41 with low thickness. This makes the tip portion 15 thin in diameter while ensuring the necessary strength and heat-radiating function.

By forming the thin portion 41 as mentioned above, image pick-up unit 31A for observation with the normal light is adjacently arranged to the image pick-up unit 31B for observation with the fluorescent light. Therefore, the cleaning performance is improved, as will be described later.

Referring to Fig. 1, the signal cables 38a and 38b are inserted in the universal cable 13 from the operating portion 12, and are connected to a relay substrate (switching unit) 42 arranged in the connector 14, switchably to a common signal cable 43.

The common signal cable 43 is connected to the video processor 4 of the external device via a scope cable 44 connected to the connector 14.

The video processor 4 comprises: CCD driving circuits 45a and 45b for driving the CCDs 35a and 35b, respectively; a signal processing circuit 46 which processes an image pick-up signal that is outputted from the CCD 35a or 35b via the relay substrate 42; and a control circuit 47 which controls the operating state of the signal processing circuit 46.

The operating portion 12 in the endoscope 2 has control switches 48a and 48b which are connected to the control circuit 47 in the video processor 4 via signal lines 49a and 49b, respectively. In this case, the control switch 48a generates a signal for instructing the switching operation, for example, and the control switch 48b generates a signal for instructing the freeze operation.

The user operates the control switch 48a, for example, thereby controlling the switching operation with a relay (switch) (shown by a dotted line in Fig. 1) of the relay substrate 42 so that the connection to the common signal cable 43 is switched to one of the signal cables 38a and 38b connected to the CCDs 35a and 35b from the other.

Reference number 49c denotes a switching signal line. A switching control terminal T in the relay substrate 42 connected to the switching signal line 49c is pulled down so as to be at the L level by resistor R. In this state, the signal cable 38a of the image pick-up unit 31A for observation with the normal light is connected to the common signal cable 43. In the starting state, the switching control terminal T is set to the L level. In other words, after the endoscope device is started, it is set in the state of observation with the normal light.

In this case, the user operates the control switch 48a, then, a signal at the H level is applied to the switching control terminal T of the relay substrate 42 via the switching signal line 49c, and the relay is switched. Further, the user operates the control switch 48a and thus the signal at the L level is applied to the switching control terminal T.

As mentioned above, the user operates the control switch 48a, then, the control circuit 47 transmits a control signal to the control circuit 58 in the light source device 3 via a control signal line 49d, and the control circuit 58 controls the generating state of excitation light for observation with the normal light or for observation with the fluorescent light in accordance with the control signal. Further, the control circuit 47 controls the operating state of the signal processing circuit 46 in accordance with the CCD 35a or 35b.

The light source device 3 comprises: a lamp 51 which generates white light including the wavelength of the excitation light; a collimator lens 52 which transfers light from the lamp 51 to a parallel luminous flux; a rotating filter 53 which is arranged in the optical path of the collimator lens 52 and having, in the circumferential direction, R, G, and B filters passing through light of R, G, and B wavelength bands within the visible light wavelength band (380 to 780 nm); a condenser lens 54 which condenses transmitting light from the rotating filter 53 and supplies the condensed light to the light guide connector 22.

The rotating filter 53 has, outside the circumferential direction in which the R, G, and B filters are arranged, a filter for excitation light which passes through the excitation light. A motor 55 rotates the rotating filter 53, and further the motor 55 is attached to a rack 56. A motor 57 with a gear engaged with the rack 56 moves the motor 55 in the direction orthogonal to an illumination light path as shown by an arrow.

The control circuit 58 controls the motor 57 with the gear. Further, the control circuit 58 is connected to the control circuit 47 of the video processor 4 via the control signal line 49d, thereby performing the corresponding control operation with the operation of the control switch 48a.

According to the embodiment, referring to Fig. 3, a fluid discharging nozzle 60 for discharging fluid such as water or air is arranged with a discharge port directed to the image pick-up unit 31A for observation with the normal light and the image pick-up unit 31B for observation with the fluorescent light which are adjacently arranged in the left and right directions.

As mentioned above, as one feature of the embodiment, the image pick-up unit 31A for observation with the normal light is arranged adjacently to the fluid discharging nozzle 60, along the air/water discharge direction of the fluid discharging nozzle 60. Further, on the discharge direction, the image pick-up unit 31B for observation with the fluorescent light is arranged adjacently to the image pick-up unit 31A for observation with the normal light.

In the present embodiment, the lines connected to this fluid discharging nozzle 60 joins into one at the tip portion thereof but branches into an air feed line 61a and a water feed line 61b in the tip portion 15.

Referring to Fig. 1, the air feed line 61a and the water feed line 61b (which are simply shown by one component of reference numeral 61 in Fig. 1) are connected to the air/water feed device (fluid supplying device) 6 including a pump (not shown) for feeding the air and water via the connector 14.

As shown in Fig. 1, an air/water feed button 63 is inserted in the air feed line 61a and the water feed line 61b, at the operating portion 12 as the halfway thereof. By operating the air/water feed button 63, fluid such as air and water can be discharged.

Thus, the fluid such as air and water is discharged, thereby making it possible that the air and the cleaning water are sprayed to the outer surface of the objective optical members 33a and 33b arranged in the discharge direction of the one fluid discharging nozzle 60 (upwards in Fig. 2 and leftwards in Fig. 3), the body fluid or the adhesion or the like is removed or washed away, and the image is picked up in the clean state or the field of view for observation is ensured.

In this case, according to the embodiment, referring to Fig. 3, the objective optical member 33a is arranged in the center of the tip surface, and the fluid discharging nozzle 60 is adjacently arranged to the left of the objective optical member 33a. Meanwhile, the objective optical member 33b is cleaned by the air and water which are discharged from the fluid discharging nozzle 60 via the outer surface of the objective optical member 33a.

That is, for the purpose of observation with the normal light, the outer surface of the objective optical member 33a is cleaner than that of the objective optical member 33b for observation with the fluorescent light because the objective optical member 33a is more frequently used than the objective optical member 33b. Therefore, the above-mentioned cleaning functions are set for the observation.

The tip side of the bending piece at the farthest tip is fixed to the back end of the outer circumference of the tip structuring member 23 shown in Fig. 3, and the outer circumference thereof is covered with a bending rubber tube.

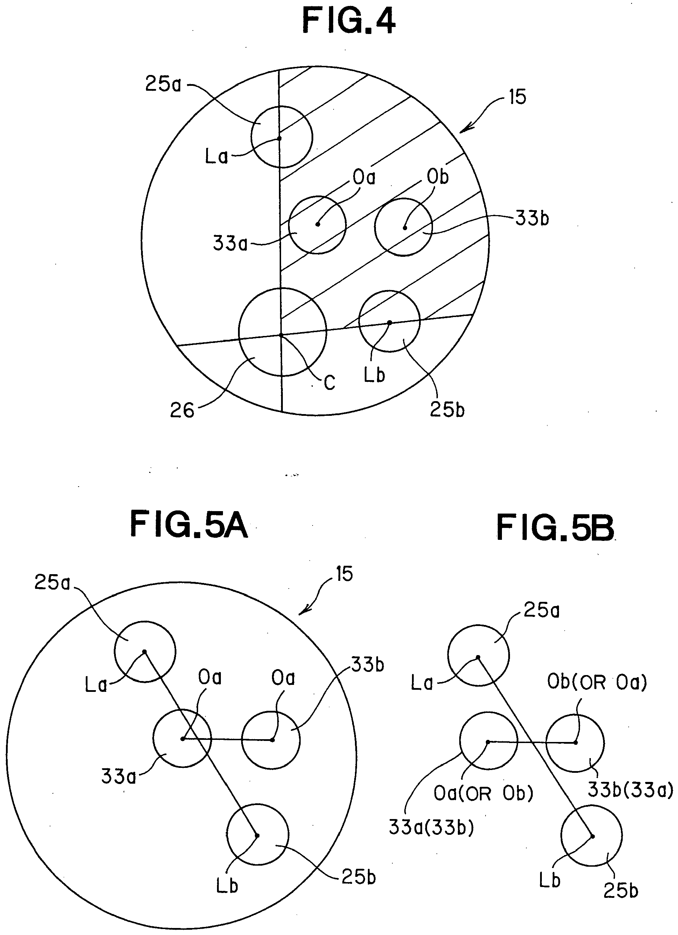

Referring to Fig. 3, reference numerals Oa and Ob denote the centers of the objective optical members 33a and 33b, reference numerals La and Lb denote the centers of the illumination optical members 25a and 25b, and reference numerals C and N denote the centers of the channel tip opening 26 and the fluid discharging nozzle 60, respectively. In Fig. 4 and other sequent diagrams, they are similar. According to the embodiment, referring to Fig. 3, an inclined portion 65 is arranged between the fluid discharging nozzle 60 and the objective optical member 33a on the tip cover 24 having the fluid discharging nozzle 60 and the like. The inclined portion 65 is extended to the up and down direction substantially orthogonal to the discharge direction of the fluid discharging nozzle 60. The inclined portion 65 reaches the edge of the channel tip opening 26 which is arranged near the bottom side of the fluid discharging nozzle 60 and the objective optical member 33a.

That is, as shown by a circle B in the enlarged view of Fig. 3, the tip cover 24 has a low portion 24L on the fluid discharging nozzle 60 side, and has a high portion 24H on the objective optical member 33a side via the inclined portion 65. In addition to the inclined portion 65, the low portion 24L and the high portion 24H reach the edge of the channel tip opening 26.

With the above-mentioned structure, when the water is fed from the fluid discharging nozzle 60 and is discharged, around the surface of the tip cover 24 on the objective optical member 33a side, the cleaning water which is not used for the cleaning is guided to the channel tip opening 26, particularly, on the down side. Consequently, a preferable field of view for observation is ensured.

In this case, the suction operation may be performed from the channel tip opening 26 by operating the suction means. Then, unnecessary cleaning water is more effectively sucked and removed.

According to the embodiment, as mentioned above, the objective optical member 33a with high using frequency (namely, the image pick-up unit 31A) is arranged near the center of the tip portion 15 of the insertion portion 11. The objective optical member 33b with low using frequency (namely, the image pick-up unit 31B) is arranged around the periphery of the tip portion 15. Thus, the life of the signal cable 38a is long.

That is, the insertion portion 11 is inserted in the bent body cavity, and is bent and is used by bending the bending portion 16. When the objective optical member 33a is arranged near the center of the insertion portion 11, the fatigue due to the distortion applied to the signal cable 38a is reduced and the long life is obtained.

The operation with the above-mentioned structure will be described.

The connector 14 of the endoscope 2 as shown in Fig. 1 is connected to the light source device 3. The connector 14 is connected to the video processor 4 via the scope cable 44. The air feed line 61a and the water feed line 61b are connected to the fluid supplying device 6.

A power switch of the external device such as the light source device 3 and the like is turned on, thereby setting the components to the operating state. In this case, the video processor 4, and the control circuits 47 and 58 in the light source device 3 receive and transmit the control signals.

In the starting state, the relay substrate 42 selects the image pick-up unit 31A for observation with the normal light. The control circuit 47 controls the operation for setting the state of observation with the normal light. That is, the control circuit 47 transmits the control signal to the control circuit 58 of the light source device 3, and sets the feeding state of the illumination light for the purpose of the observation with the normal light.

Further, the control circuit 47 controls the operation for driving the CCD driving circuit 45a, and sets the operating state of the signal processing circuit 46 to the observation mode with the normal light.

An operator inserts the insertion portion 11 of the endoscope 2 in the body cavity, and sets the operation for observing the affected part as the diagnostic target.

The light source device 3 enters the feeding state of the illumination light for observation with the normal light. In this state, the rotating filter 53 is rotated by the motor 55 while the R, G, and B filters are arranged in the illumination light path. The R, G, and B illumination light is supplied to the light guide 21 in the order of surface sequence. Synchronously to this, the CCD driving circuit 45a outputs the CCD driving signal, and illuminates the affected part in the body cavity via the illumination optical members 25a and 25b.

An image of an illuminated subject of the affected part is formed to the light receiving surface of the CCD 35a through the objective optical member 33a of the image pick-up unit 31A for observation with the normal light, and is photoelectrically converted. The CCD 35a outputs the photoelectrically-converted signal by applying the CCD driving signal. The output signal passes through the signal cable 38a and the common signal cable 43 selected by the relay substrate 42, and is inputted to the signal processing circuit 46.

The signal inputted to the signal processing circuit 46 is A/D-converted. Then, the signal is temporarily stored in memories for R, G, and B.

Subsequently, the signals stored in the memories for R, G, and B are simultaneously read and, then, become synchronized R, G, and B signals. Further, the signals are D/A-converted and then become analog signals R, G, and B. These signals are displayed as colors on the monitor 5.

When the operator checks the affected part in detail by observation with the fluorescent light in addition to observation with the normal light, he/she switches on the control switch 48a. Then, the control circuit 47 receives a switching instructing signal and controls the switching operation of the relay substrate 42. Further, the light source device 3 is set via the control circuit 58 to supply the excitation light for observation with the fluorescent light.

The control circuit 47 controls the CCD driving circuit 45b to the operating state, and further sets the signal processing circuit 46 to a processing mode for observation with the fluorescent light.

In this case, the control circuit 58 in the light source device 3 moves, by using the motor 57 with gear, the rotating filter 53 together with the motor 55 in the direction orthogonal to the illumination light path, thus to arrange the excitation light filter in the illumination light path.

In this case, among the light from the lamp 51, the excitation light filter passes through the light having the wavelength band near 400 to 450 nm, and the passing light is supplied to the light guide 21. The excitation light is irradiated to the affected part in the body cavity via the illumination optical members 25a and 25b.

When the affected part or the like irradiated with the excitation light is the cancer organ, it absorbs the excitation light and radiates the fluorescent light which is stronger than that of the normal organ (with autofluorescence). The image of the light at the portion irradiating the fluorescent light is formed to the light receiving surface of the CCD 35b through the objective optical member 33b in the image pick-up unit 31B for observation with the fluorescent light.

The CCD 35b outputs the photoelectrically-converted signal by applying the CCD driving signal from the CCD driving circuit 45b. In this case, the signal is amplified in the CCD 35b, and is outputted from the CCD 35b. The signal passes through the signal cable 38b and the common signal cable 43 selected by the relay substrate 42, and is inputted to the signal processing circuit 46. The signal inputted to the signal processing circuit 46 is A/D-converted therein and then is simultaneously stored in the memories for R, G, and B.

Subsequently, the signals stored in the memories for R, G, and B are simultaneously read and then become synchronized signals R, G, and B. Further, the signals are D/A-converted and then become analog signals R, G, and B. Finally, the signals are displayed as monochrome colors on the monitor 5.

Incidentally, the signals may be displayed as pseudo colors by comparing the signal levels inputted to the signal processing circuit 46 with a plurality of thresholds and then by changing assigned colors in accordance with the comparison result. According to the embodiment, the observation is performed with the normal light and further the observation is performed with the fluorescent light. Therefore, as compared with the endoscope only for observation with the normal light, the endoscope enables the easy diagnosis. Further, according to the embodiment, the image pick-up units 31A and 31B for observation with the normal light and the fluorescent light are arranged and therefore it is possible to obtain both preferable observed images with the normal light and the fluorescent light.

Specifically, the image pick-up operation with the fluorescent light needs to the image pick-up operation with the light which is weaker than the light in observation with the normal light, and further needs the higher S/N ratio. When the image pick-up operation with the fluorescent light shares with the normal CCD, the image is liable to lower S/N ratio. However, according to the embodiment, the dedicated CCD 35b is used for the image pick-up operation with the fluorescent light and therefore the image with the fluorescent light is obtained with the preferable S/N ratio.

The relay substrate 42 is arranged for the switching operation and one of the image pick-up units 31A and 31B is connected to the video processor 4. Thus, the compact endoscope system 1 is formed, as compared with the case of always driving the two image pick-up units 31A and 31B and processing the signals thereof.

According to the embodiment, the one fluid discharging nozzle 60 sprays the cleaning water and air to the outer surfaces of both the objective optical members 33a and 33b, and the objective optical members 33a and 33b are set to the clean state, thus to ensure the preferable field of view for observation. As a consequence, the insertion portion 11 has a thinner diameter and the pain for the patient is reduced upon inserting the endoscope, and the application range capable of inserting the endoscope can be enlarged.

The endoscope 2 according to the embodiment has the appearance structure of the existing endoscope having only the image pick-up unit for observation with the normal light. Further, the endoscope 2 according to the embodiment is connected, via the scope cable 44, to a video processor (not shown) which drives the existing endoscope having only the image pick-up unit for observation with the normal light and which performs the signal processing thereof and, thus, the endoscope 2 is used as the endoscope for observation with the normal light, similarly to the existing endoscope.

That is, the endoscope 2 according to the embodiment keeps the compatibility to the existing endoscope having only the image pick-up unit for observation with the normal light, and is used by connection to the existing video processor. In this case, it is also possible to adapt so as not to select the observation with the fluorescent light by the image pick-up unit 31B in the relay substrate 42.

Further, the endoscope 2 according to the embodiment has the structure having various advantages, as will be described hereinbelow.

According to the embodiment, the objective optical member 33a (namely, image pick-up unit 31A) and the objective optical member 33b (namely, image pick-up unit 31B) are arranged in the left and right directions approximately at the center in the up and down directions.

The bending portion 16 is set so that the bending amount in the up and down directions is larger than that in the left and right directions. In the case of bending, the inside of the bending portion and the outside thereof mutually receive force in the opposite direction, and the center of the bending portion is little influenced by the opposite force.

Therefore, the above-mentioned arrangement prevents the reduction of the life of the signal cables 38a and 38b due to the fatigue caused by the iterative bending operation.

Referring to Figs. 2 and 3, the image pick-up unit 31B is arranged to the peripheral side of the tip portion 15 of the insertion portion 11, apart from the center of the tip portion 15. The image pick-up unit 31B uses the CCD 35b with the amplifying structure therein as mentioned above, and has the thermal tolerance lower than that of the CCD 35a. Therefore, by arranging the image pick-up unit 31B on the peripheral side of the tip portion 15 apart from the center thereof, the heat generated in the tip portion 15 is effectively radiated, as compared with the case of arranging the image pick-up unit 31B in the center of the tip portion 15 where the heat is not easily radiated.

Referring to Fig. 3, the image pick-up unit 31B is arranged apart from the illumination optical members 25a and 25b so as to prevent the influence by the heat generated by the light guide 21. Similarly, the image pick-up unit 31A is arranged relatively apart from the illumination optical members 25a and 25b so as to prevent the influence by the heat generated by the light guide 21.

Referring to Fig. 2, the protecting tube 39b covers the signal cable 38b in the image pick-up unit 31B arranged apart from the center of the tip portion 15, and the thickness of the protecting tube 39b is thinner than that of the protecting tube 39a for covering the signal cable 38a in the image pick-up unit 31A arranged near the center of the tip portion 15, and thus facilitating bending operation. That is, the amount of bending operation force is reduced.

Further, the positions of the light receiving surfaces 40a and 40b of the CCDs 35a and 35b are deviated in the longitudinal direction of the tip portion 15, thereby suppressing the influence by the heat generated in the CCDs 35a and 35b, as compared with the positions matching each other. That is, since the heating positions are different, the generated heat is easily radiated as compared with the positions matching each other. The thermal influence on the other CCD is reduced.

According to the embodiment, with the arrangement as shown in Figs. 3 and 4, in the case of projecting the treatment tool from the channel tip opening 26, it is possible to prevent a state in which the projected treatment tool shades both the objective optical members 33a and 33b.

Referring to Fig. 4, in the state shown in Fig. 3, a plurality of objective optical members 33a and 33b are arranged in an area shown by a portion with slanted lines. The area is one of four areas formed by dividing with two straight lines connecting the center C of the channel tip opening 26 and the center La of the illumination optical member 25a, and the center C of the channel tip opening 26 and the center Lb of the illumination optical member 25b, respectively. The area includes both (the half of) the illumination optical members 25a and 25b.

The above-mentioned arrangement prevents the state in which the treatment tool projected from the channel tip opening 26 shades both the objective optical members 33a and 33b. Thus, in the case of using the treatment tool, the preferable field of view for observation is ensured.

As will be understood with reference to Fig. 3, the illumination optical members 25a and 25b, or the objective optical members 33a and 33b are arranged at the position where the line segment connecting the centers La and Lb of the illumination optical members 25a and 25b crosses the line segment connecting the centers Oa and Ob of the objective optical members 33a and 33b. An example of the arrangement is shown in Fig. 5A.

In the schematic diagram shown in Fig. 5B according to a modification of the embodiment, the illumination optical members 25a and 25b, or the objective optical members 33a and 33b may be arranged at the position where both the line segments cross each other near the center. Alternatively, the arrangement positions of the objective optical members 33a and 33b may be changed.

With the arrangement structure, the illumination light outputted from the illumination optical members 25a and 25b is properly distributed so that it equally illuminates the observed targets of the objective optical members 33a and 33b.

According to the embodiment or the modification thereof, the arrangement structure is set as follows. That is, in the case of switching the observation with the normal light using the objective optical member 33a and the observation with the fluorescent light using the objective optical member 33b, the change amount of the treatment tool projected from the channel tip opening 26 is small (within an angle of 90°) in the observing direction (projecting direction) (within the field of view for observation) and thus the operability is improved.

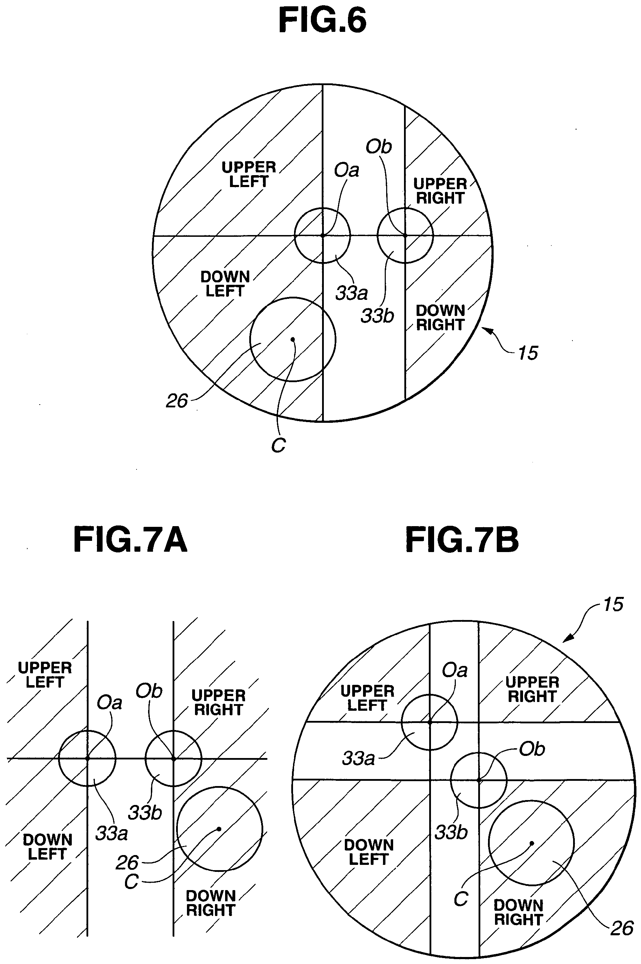

Referring to Fig. 3, center lines pass through the centers Oa and Ob of the objective optical members 33a and 33b and are orthogonal to each other in the up and down directions and in the left and right directions, and divide the area into six ones. Fig. 6 shows the six areas in this case.

Referring to Fig. 6, the center C of the channel tip opening 26 is arranged in an area with slanted lines shown by shading, namely, any of the areas in the upper left, down left, upper right, and down right directions. According to the embodiment, the center C of the channel tip opening 26 is arranged in the area in the down left direction but, alternatively, may be arranged in another area with slanted lines.

As shown in a schematic diagram of Fig. 7A, the center C of the channel tip opening 26 may be arranged in the area in the down right.

According to the embodiment, the objective optical members 33a and 33b are arranged along the horizontal direction (left and right directions). Fig. 7B shows an arrangement example according to the modification, in which the objective optical members 33a and 33b may be arranged along the direction except for the horizontal direction.

In this case, the tip portion 15 is divided into nine, by using center lines in the horizontal directions passing through the centers Oa and Ob of the objective optical members 33a and 33b and center lines orthogonal to the first center lines passing though the centers Oa and Ob.

In this case, the center C of the channel tip opening 26 is arranged in four areas with slanted lines similarly to the case in Fig. 6, namely, any of the areas in the upper left, down left, upper right, and down right. Thus, the objective optical members 33a or 33b is switched and then, advantageously, the change amount of the treatment tool projected from the channel tip opening 26 is reduced in the observing direction.

### Industrial Applicability

In case where an affected part in a body cavity or the like is examined with an endoscope, it is possible to perform color observation with the normal light by the image pick-up unit for observation with the normal light and also perform specific light observation by the image pick-up unit for observation with the specific light by the switching operation.

## Claims

1. An endoscope having an insertion portion (11) which is to be inserted into an examined body, comprising:
a first image pick-up portion (31A) provided at a distal end portion (15) of the insertion portion (11) for performing observation of a subject under a first condition, the first image pick-up portion (31A) having a first objective optical member (33a) and a first solid-state image pick-up (35a) device arranged at a position to form image of the first objective optical member (33a);
a second image pick-up portion (31B) provided at the distal end portion of the insertion portion for performing observation of the subject under a second condition, the second image pick-up portion (31B) having a second objective optical member (33b) and a second solid-state image pick-up device (35b) arranged at a position to form image of the second objective optical member (33b);
a switching device (42) for switching between an observation image of the subject picked up by the first image pick-up portion (31A) to be displayed on display means (5) and an observation image of the subject picked up by the second image pick-up portion (31B) to be displayed on the display means (5);
a nozzle (60) arranged such as to be able to discharge fluid to the first objective optical member (33a) and the second objective optical member (33b);
two illumination optical members (25a, 25b), each illumination optical member (25a, 25b) being connected to a light guide (21) so as to output illumination light to the distal end portion (15) of the insertion portion (11); and
a treatment tool channel which permits the projection of a treatment tool from an opening (26) arranged to the distal end portion (15) of the insertion portion (11);
**characterized in that**
the tips of the light guides (21) are fixed to the inside of illumination windows of a tip structuring member (23) forming the distal end portion (15) and the illumination optical members (25a, 25b) are attached right in front of the light guides (21),
wherein the distal end portion (15) presents a tip cover (24) on which the nozzle (60) is mounted, the tip cover (24) presenting:
an inclined portion (65) arranged between the nozzle (60) and the first objective optical member (33a) on the tip cover (24);
a low portion (24L) on the side of the nozzle (60); and
a high portion (24H) on the side of the first objective optical member (33a) via the inclined portion (65),
wherein said inclined portion (65) reaches the edge of the channel tip opening (26) which is arranged near a bottom side of the nozzle (60) and the first objective optical member (33a).

2. The endoscope according to Claim 1, **characterized in that** the nozzle (60) has an opening for discharging fluid substantially in one direction, and the first and second objective optical members (33a, 33b) are arranged in the one direction.

3. The endoscope according to Claim 1, **characterized in that** the first image pick-up portion (31A) is an image pick-up portion for observation with normal light for performing observation of the subject with the normal light and the second image pick-up portion (31B) is an image pick-up portion for observation with specific light for performing observation of the subject with the specific light, and
the first objective optical member (33a) of the first image pick-up portion (31A) is arranged nearer to the nozzle (60) than the second objective optical member (33b) of the second image pick-up portion (31B).

4. The endoscope according to Claim 1, **characterized in that** the first and second objective optical members (33a, 33b) are arranged in an area surrounded by two straight lines which are extended from the center (C) of the opening (26) towards the centers (La, Lb) of the two illumination optical members (25a, 25b) respectively and the outer periphery of the distal end surface (15) of the insertion portion (11) at the distal end surface of the distal end portion (15) of the insertion portion (11).

5. The endoscope according to Claim 3, **characterized in that** the distal end portion (15) of the insertion portion (11) has two illumination optical members (25a, 25b) capable to output illumination light, and the illumination optical members (25a, 25b) or the objective optical members (33a, 33b) are arranged so that a line segment connecting the centers (La, Lb) of the two illumination optical members crosses a line segment connecting the centers (Oa, Ob) of the first and second objective optical members (33a, 33b).

6. The endoscope according to Claim 3, **characterized in that** the image pick-up portion (31B) for observation with the specific light is an image pick-up portion for observation with fluorescent light for obtaining a fluorescent image of the subject.

7. The endoscope according to Claim 6, **characterized in that** the image pick-up portion for observation with the fluorescent light (31B) has an excitation light cut-off filter capable to cut-off excitation light.

8. The endoscope according to Claim 1, **characterized in that** the switching device (42) is provided to a connector for detachably connecting the endoscope to an external device which is used in combination with the endoscope.

9. The endoscope according to Claim 1, **characterized in that** the second solid-state image pick-up (35b) device is a solid-state image pick-up device having an amplifying function therein.

10. The endoscope according to Claim 1, **characterized in that** the inclined portion (65) is configured so as to permit guiding of fluid discharged by the nozzle (60) to the opening (26) and sucking of fluid through the opening (26).

## Patentansprüche

1. Endoskop mit einem Einführabschnitt (11), der dazu vorgesehen ist, in einen untersuchten Körper eingeführt zu werden, umfassend:
einen ersten Bildaufnahmeabschnitt (31A), der an einem distalen Endabschnitt (15) des Einführabschnitts (11) vorgesehen ist, um eine Beobachtung eines Subjekts unter einer ersten Bedingung durchzuführen, wobei der erste Bildaufnahmeabschnitt (31A) ein erstes optisches Objektivelement (33a) und eine erste Halbleiter-Bildaufnahmeeinrichtung (35a) umfasst, die an einer Position angeordnet ist, um ein Bild des ersten optischen Objektivelements (33a) zu bilden;
einen zweiten Bildaufnahmeabschnitt (31B), der an dem distalen Endabschnitt des Einführabschnitts vorgesehen ist, um eine Beobachtung des Subjekts unter einer zweiten Bedingung durchzuführen, wobei der zweite Bildaufnahmeabschnitt (31B) ein zweites optisches Objektivelement (33b) und eine zweite Halbleiter-Bildaufnahmeeinrichtung (35b) umfasst, die an einer Position angeordnet ist, um ein Bild des zweiten optischen Objektivelements (33b) zu bilden;
eine Schalteinrichtung (42) zum Umschalten zwischen einem von dem ersten Bildaufnahmeabschnitt (31A) aufgenommenen Beobachtungsbild des Subjekts, das zur Anzeige auf einem Anzeigemittel (5) vorgesehen ist, und einem von dem zweiten Bildaufnahmeabschnitt (31B) aufgenommenen Beobachtungsbild des Subjekts, das zur Anzeige auf dem Anzeigemittel (5) vorgesehen ist;
eine Düse (60), die so angeordnet ist, dass sie dazu in der Lage ist, ein Fluid zu dem ersten optischen Objektivelement (33a) und dem zweiten optischen Objektivelement (33b) abzugeben;
zwei optische Beleuchtungselemente (25a, 25b), wobei jedes optische Beleuchtungselement (25a, 25b) mit einem Lichtleiter (21) verbunden ist, um dem distalen Endabschnitt (15) des Einführabschnitts (11) Beleuchtungslicht auszugeben; und
einen Behandlungsinstrumentenkanal, der das Vorschieben eines Behandlungsinstruments von einer Öffnung (26) erlaubt, die zu dem distalen Endabschnitt (15) des Einführabschnitts (11) angeordnet ist;
**dadurch gekennzeichnet, dass**
die Spitzen der Lichtleiter (21) an der Innenseite von Beleuchtungsfenstern eines Spitzenstrukturierungselements (23), das den distalen Endabschnitt (15) bildet, befestigt sind und die optischen Beleuchtungselemente (25a, 25b) genau vor den Lichtleitern (21) befestigt sind,
wobei der distale Endabschnitt (15) eine Spitzenabdeckung (24) darstellt, an der die Düse (60) montiert ist, wobei die Spitzenabdeckung (24) darstellt:
einen geneigten Abschnitt (65), der zwischen der Düse (60) und dem ersten optischen Objektivelement (33a) an der Spitzenabdeckung (24) angeordnet ist;
einen niedrigen Abschnitt (24L) auf der Seite der Düse (60); und
einen hohen Abschnitt (24H) auf der Seite des ersten optischen Objektivelements (33a) über den geneigten Abschnitt (65),
wobei der geneigte Abschnitt (65) den Rand der Kanalspitzenöffnung (26) erreicht, der in der Nähe einer Unterseite der Düse (60) und des ersten optischen Objektivelements (33a) angeordnet ist.

2. Endoskop nach Anspruch 1, **dadurch gekennzeichnet, dass** die Düse (60) eine Öffnung zum Ausgeben eines Fluids im Wesentlichen in einer Richtung hat und die ersten und zweiten optischen Objektivelemente (33a, 33b) in der einen Richtung angeordnet sind.

3. Endoskop nach Anspruch 1, **dadurch gekennzeichnet, dass** der erste Bildaufnahmeabschnitt (31A) ein Bildaufnahmeabschnitt zur Beobachtung mit normalem Licht zur Durchführung einer Beobachtung des Subjekts mit normalem Licht und der zweite Bildaufnahmeabschnitt (31B) ein Bildaufnahmeabschnitt zur Beobachtung mit spezifischen Licht zur Durchführung einer Beobachtung des Subjekts mit dem spezifischen Licht ist, und
das erste optische objektive Element (33a) des ersten Bildaufnahmeabschnitts (31A) näher an der Düse (60) angeordnet ist als das zweite optische objektive Element (33b) des zweiten Bildaufnahmeabschnitts (31B).

4. Endoskop nach Anspruch 1, **dadurch gekennzeichnet, dass** die ersten und zweiten optischen Objektivelemente (33a, 33b) in einem Bereich angeordnet sind, der von zwei geraden Linien, welche sich von einem Mittelpunkt (C) der Öffnung (26) in Richtung der Mittelpunkte (La, Lb) der jeweiligen zwei optischen Beleuchtungselemente (25a, 25b) erstrecken, und dem Außenumfang der distalen Endfläche (15) des Einführabschnitts (11) an der distalen Endfläche des distalen Endabschnitts (15) des Einführabschnitts (11) umgeben ist.

5. Endoskop nach Anspruch 3, **dadurch gekennzeichnet, dass** der distale Endabschnitt (15) des Einführabschnitts (11) zwei optische Beleuchtungselemente (25a, 25b) hat, die dazu in der Lage sind, Beleuchtungslicht auszugeben, und die optischen Beleuchtungselemente (25a, 25b) oder die optischen Objektivelemente (33a, 33b) so angeordnet sind, dass ein Liniensegment, das die Mittelpunkte (La, Lb) der zwei optischen Beleuchtungselemente verbindet, ein Liniensegment kreuzt, das die Mittelpunkte (Oa, Ob) der ersten und zweiten optischen Objektivelemente (33a, 33b) verbindet.

6. Endoskop nach Anspruch 3, **dadurch gekennzeichnet, dass** der Bildaufnahmeabschnitt (31B) zur Beobachtung mit dem spezifischen Licht ein Bildaufnahmeabschnitt zur Beobachtung mit Fluoreszenzlicht ist, um ein Fluoreszenzbild des Subjekts zu erhalten.

7. Endoskop nach Anspruch 6, **dadurch gekennzeichnet, dass** der Bildaufnahmeabschnitt zur Beobachtung mit dem Fluoreszenzlicht (31B) einen Anregungslicht-Abschirmfilter hat, der dazu in der Lage ist, Anregungslicht abzuschirmen.

8. Endoskop nach Anspruch 1, **dadurch gekennzeichnet, dass** die Schalteinrichtung (42) an einem Verbinder zum lösbaren Verbinden des Endoskops mit einer externen Einrichtung, die in Kombination mit dem Endoskop verwendet wird, vorgesehen ist.

9. Endoskop nach Anspruch 1, **dadurch gekennzeichnet, dass** die zweite Halbleiter-Bildaufnahmeeinrichtung (35b) eine Halbleiter-Bildaufnahmeeinrichtung ist, die eine Verstärkungsfunktion hat.

10. Endoskop nach Anspruch 1, **dadurch gekennzeichnet, dass** der geneigte Abschnitt (65) dazu eingerichtet ist, das Führen eines von der Düse (60) abgegebenen Fluids zu der Öffnung (60) und das Saugen des Fluids durch die Öffnung (60) zu erlauben.

## Revendications

1. Endoscope comportant une partie d'insertion (11) qui doit être insérée dans un corps examiné, comprenant :
une première partie (31A) de capture d'image prévue au niveau d'une partie d'extrémité distale (15) de la partie d'insertion (11) pour réaliser une observation d'un sujet sous une première condition, la première partie (31A) de capture d'image comportant un premier élément optique d'objectif (33a) et un premier dispositif (35a) de capture d'image à l'état solide agencé au niveau d'une position pour former une image du premier élément optique d'objectif (33a) ;
une deuxième partie (31B) de capture d'image prévue au niveau de la partie d'extrémité distale de la partie d'insertion pour réaliser une observation du sujet sous une deuxième condition, la deuxième partie (31B) de capture d'image comportant un deuxième élément optique d'objectif (33b) et un deuxième dispositif (35b) de capture d'image à l'état solide agencé au niveau d'une position pour former une image du deuxième élément optique d'objectif (33b) ;
un dispositif de commutation (42) destiné à commuter entre une image d'observation du sujet capturée par la première partie (31A) de capture d'image devant être affichée sur un moyen d'affichage (5) et une image d'observation du sujet capturée par la deuxième partie (31B) de capture d'image devant être affichée sur le moyen d'affichage (5) ;
une buse (60) agencée de façon à être capable d'évacuer un fluide vers le premier élément optique d'objectif (33a) et le deuxième élément optique d'objectif (33b) ;
deux éléments optiques d'éclairage (25a, 25b), chaque élément optique d'éclairage (25a, 25b) étant connecté à un guide de lumière (21) de façon à délivrer en sortie une lumière d'éclairage vers la partie d'extrémité distale (15) de la partie d'insertion (11) ; et
un canal d'outil de traitement qui permet la projection d'un outil de traitement depuis une ouverture (26) agencée sur la partie d'extrémité distale (15) de la partie d'insertion (11) ;
**caractérisé en ce que**
les pointes des guides de lumière (22) sont fixées à l'intérieur des fenêtres d'éclairage d'un élément (23) structurant une pointe formant la partie d'extrémité distale (15) et les éléments optiques d'éclairage (25a, 25b) sont attachés juste en face des guides de lumière (21),
dans lequel la partie d'extrémité distale (15) présente un embout (24) sur lequel la buse (60) est montée, l'embout (24) présentant :
une partie inclinée (65) agencée entre la buse (60) et le premier élément optique d'objectif (33a) sur l'embout (24) ;
une partie inférieure (24L) sur le côté de la buse (60) ; et
une partie supérieure (24H) sur le côté du premier élément optique d'objectif (33a) via la partie inclinée (65),
dans lequel ladite partie inclinée (65) atteint le bord de l'ouverture (26) de pointe de canal qui est agencée près d'un côté inférieur de la buse (60) et du premier élément optique d'objectif (33a).

2. Endoscope selon la revendication 1, **caractérisé en ce que** la buse (60) comporte une ouverture destinée à évacuer du fluide sensiblement dans une direction, et les premier et deuxième éléments optiques d'objectif (33a, 33b) sont agencés dans cette direction.

3. Endoscope selon la revendication 1, **caractérisé en ce que** la première partie (31A) de capture d'image est une partie de capture d'image pour observation avec une lumière normale destinée à réaliser une observation du sujet avec la lumière normale et la deuxième partie (31B) de capture d'image est une partie de capture d'image pour observation avec une lumière spécifique destinée à réaliser une observation du sujet avec la lumière spécifique, et
le premier élément optique d'objectif (33a) de la première partie (31A) de capture d'image est agencé plus près de la buse (60) que le deuxième élément optique d'objectif (33b) de la deuxième partie (31B) de capture d'image.

4. Endoscope selon la revendication 1, **caractérisé en ce que** les premier et deuxième éléments optiques d'objectif (33a, 33b) sont agencés dans une zone entourée par deux lignes droites qui s'étendent depuis le centre (C) de l'ouverture (26) vers les centres (La, Lb) des deux éléments optiques d'éclairage (25a, 25b) respectivement et la périphérie externe de la surface d'extrémité distale (15) de la partie d'insertion (11) au niveau de la surface d'extrémité distale de la partie d'extrémité distale (15) de la partie d'insertion (11).

5. Endoscope selon la revendication 3, **caractérisé en ce que** la partie d'extrémité distale (15) de la partie d'insertion (11) comporte deux éléments optiques d'éclairage (25a, 25b) capables de délivrer en sortie une lumière d'éclairage, et les éléments optiques d'éclairage (25a, 25b) ou les éléments optiques d'objectif (33a, 33b) sont agencés de sorte qu'un segment de ligne reliant les centres (La, Lb) des deux éléments optiques d'éclairage croise un segment de ligne reliant les centres (Oa, Ob) des premier et deuxième éléments optiques d'objectif (33a, 33b).

6. Endoscope selon la revendication 3, **caractérisé en ce que** la partie (31B) de capture d'image pour observation avec la lumière spécifique est une partie de capture d'image pour observation avec une lumière fluorescente destinée à obtenir une image fluorescente du sujet.

7. Endoscope selon la revendication 6, **caractérisé en ce que** la partie de capture d'image pour observation avec la lumière fluorescente (31B) comporte un filtre de coupure de lumière d'excitation capable de couper la lumière d'excitation.

8. Endoscope selon la revendication 1, **caractérisé en ce que** le dispositif de commutation (42) est prévu sur un connecteur destiné à connecter de manière détachable l'endoscope à un dispositif externe qui est utilisé en combinaison avec l'endoscope.

9. Endoscope selon la revendication 1, **caractérisé en ce que** le deuxième dispositif (35b) de capture d'image à l'état solide est un dispositif de capture d'image à l'état solide ayant une fonction d'amplification dans celui-ci.

10. Endoscope selon la revendication 1, **caractérisé en ce que** la partie inclinée (65) est configurée de façon à permettre le guidage du fluide évacué par la buse (60) vers l'ouverture (26) et l'aspiration du fluide à travers l'ouverture (26).
